# EUROPÄISCHE PATENTSCHRIFT

(11) **EP 4 123 259 B1**
(45) Veröffentlichungstag und Bekanntmachung des Hinweises auf die Patenterteilung: **13.12.2023**
(21) Anmeldenummer: 22186269.1
(22) Anmeldetag: 21.07.2022
(51) Int. Cl.: G01B 15/04, G01B 21/04, A01C 1/02, B33Y 80/00, G01N 33/00, G01N 23/046

(54) **VERFAHREN ZUR ÜBERWACHUNG UND/ODER KALIBRIERUNG EINER EINRICHTUNG, DIE ZUR DREIDIMENSIONALEN RÖNTGENOPTISCHEN ÜBERPRÜFUNG VON KEIMLINGEN IN VERSCHIEDENEN WACHSTUMSPHASEN AUSGEBILDET IST**
METHOD FOR MONITORING AND/OR CALIBRATING A DEVICE DESIGNED FOR THREE-DIMENSIONAL X-RAY EXAMINATION OF SEEDLINGS IN DIFFERENT GROWTH PHASES
PROCÉDÉ DE SURVEILLANCE ET/OU D'ÉTALONNAGE D'UN DISPOSITIF CONÇU POUR VÉRIFIER PAR RAYONS X EN TROIS DIMENSIONS DES GERMES PENDANT DIFFÉRENTES PHASES DE CROISSANCE

(30) Priorität: 23.07.2021 DE 102021207924
(43) Veröffentlichungstag der Anmeldung: 25.01.2023
(73) Patentinhaber: Fraunhofer-Gesellschaft zur Förderung der angewandten Forschung e.V., 80686 München (DE); Strube D&S GmbH, 38387 Söllingen (DE); Wolff, Antje, 23669 Timmendorfer Strand (DE)
(72) Erfinder: Porsch, Felix, 66123 Saarbrücken (DE); Wolff, Antje, 38387 Söllingen (DE); Gelz, Andreas, 66123 Saarbrücken (DE); Götz, Yvonne, 38387 Söllingen (DE); Neuhoff, Marc, 38387 Söllingen (DE)
(74) Vertreter: Pfenning, Meinig & Partner mbB

(56) Entgegenhaltungen:
- EP-B1- 2 525 641
- CN-A- 106 709 986
- WOLFGANG H STUPPY ET AL: "Three-dimensional analysis of plant structure using high-resolution X-ray computed tomography", TRENDS IN PLANT SCIENCE, Bd. 8, Nr. 1, 2003, Seiten 2-6, XP055137776, ISSN: 1360-1385, DOI: 10.1016/S1360-1385(02)00004-3
- S. D. MALLETT ET AL: "Additive manufacturing and computed tomography of bio-inspired anchorage systems", GéOTECHNIQUE LETTERS, Bd. 8, Nr. 3, September 2018 (2018-09), Seiten 219-225, XP055707578, GB ISSN: 2045-2543, DOI: 10.1680/jgele.18.00090

## Beschreibung

Die Erfindung betrifft ein Verfahren zur Überwachung und/oder Kalibrierung einer Einrichtung, die zur dreidimensionalen röntgenoptischen Überprüfung von Keimlingen in verschiedenen Wachstumsphasen ausgebildet ist.

Zunehmend halten moderne Techniken Einzug in den eher konservativen Bereich der Agrartechnik. Dazu gehört auch der Einsatz von Computertomographie (CT) zur Qualitätskontrolle bei der Produktion von Saatgut.

Zur Qualitätskontrolle gehört auch, dass das jeweilige Prüfgerät selbst regelmäßig kontrolliert wird. Eine übliche Methode dafür ist die Verwendung von Referenzproben und Qualitätsregelkarten. Die Referenzproben werden regelmäßig mit dem Prüfgerät geprüft und die Messergebnisse in die Regelkarte eingetragen. So können im Laufe der Zeit auftretende systematische Veränderungen von Messwerten erkannt und ggf. Korrekturmaßnahmen ergriffen werden.

Eine wesentliche Voraussetzung dabei ist es, dass sich die jeweilige Referenzprobe selbst nicht im Laufe der Zeit verändert. Diese Methode wird schon seit vielen Jahren bei der Prüfung von Zuckerrübensamen mittels Computertomographie erfolgreich eingesetzt.

Ein neues Anwendungsgebiet der CT für die Qualitätssicherung in der Saatgutproduktion ist der Einsatz für die Beurteilung der Keimfähigkeit und Triebkraft von Keimlingen. Dabei werden bei sogenannten Keimtests die wachsenden Keimlinge mehrmals im Abstand von Tagen tomographiert und die CT-Aufnahmen mittels digitaler Bildverarbeitung ausgewertet (siehe z.B. EP 2525641 B1).

Bei dieser Anwendung können jedoch keine Keimlinge als Referenzprobe verwendet werden, da diese sich stetig verändern und auch nicht lange haltbar sind.

Um trotzdem eine gewisse Qualitätssicherung zu erreichen, werden verschiedene Prozessparameter ständig überwacht. Dazu gehören z.B. die Betriebswerte der Röntgenröhre oder die Kantenschärfe in der CT-Rekonstruktion. Allerdings kann damit nicht ausgeschlossen werden, dass unerkannte Störgrößen das Ergebnis beeinflussen. Auch ist der Einfluss von Störgrößen auf das Endergebnis der Auswertung nur schlecht abzuschätzen, da der Zusammenhang zwischen der Radiographieaufnahme, der CT-Rekonstruktion und der Bildauswertung nur indirekt und sehr komplex ist.

Qualitätsregelkarten können daher mit dieser Methode nicht erstellt werden. Es ist daher Aufgabe der Erfindung, Möglichkeiten anzugeben, mit denen die Überwachung und/oder Kalibrierung von Prüfgeräten oder Einrichtungen mit denen eine dreidimensionale röntgenoptische Überprüfung von Keimlingen verbessert, die Genauigkeit erhöht und vergleichbare Ergebnisse an verschiedensten Keimlingen erhalten werden können.

Erfindungsgemäß wird diese Aufgabe mit einem Verfahren, das die Merkmale des Anspruchs 1 aufweist, gelöst. Vorteilhafte Ausgestaltungen und Weiterbildungen der Erfindung können mit in abhängigen Ansprüchen bezeichneten Merkmalen realisiert werden.

Bei dem Verfahren werden natürliche Keimlinge zu vorgegebenen Zeiten während ihrer Wachstumsphase dreidimensional optisch oder röntgenoptische vermessen. Mit den erfassten Messwerten wird jeweils ein Steuerungsprogramm für eine Einrichtung, die zum dreidimensionalen Drucken künstlicher Keimlinge als Referenzproben, die den natürlichen Keimlingen nachgebildet sind, ausgebildet ist, erstellt. Die mit den entsprechend erstellten Steuerprogrammen herzustellenden künstlichen Keimlinge werden dabei einem Kunststoff hergestellt.

Die so hergestellten künstlichen Keimlinge werden dann dreidimensional röntgenoptisch vermessen. Die dabei erfassten Messwerte werden in eine Regelkarte (control chart) eingetragen oder eine bereits erstellte Regelkarte wird entsprechend angepasst. Mit der Regelkarte wird eine Überwachung und/oder Kalibrierung der Einrichtung, die zum dreidimensionalen röntgenoptischen Überprüfung von Keimlingen ausgebildet ist (insbesondere einem Computertomographen), durchgeführt.

Mit der Einrichtung, die zum dreidimensionalen Drucken ausgebildet ist, kann der jeweilige Kunststoff tropfenweise oder mittels Filamenten, bei dem so genannten Fused Filament Fabrication (FFF) oder mittels Stereolithographie verarbeitet werden, um künstliche Keimlinge herzustellen, die eine Dimensionierung und geometrische Gestalt aufweisen, die natürlichen Keimlingen in verschiedenen vorgebbaren Wachstumsphasen entsprechen.

Mit den optisch oder röntgenoptisch erfassten dreidimensionalen Messwerten von natürlichen Keimen kann jeweils ein CAD-Steuerprogramm erstellt werden, um damit die Einrichtung zum Drucken entsprechend anzusteuern.

So kann man künstliche Keimlinge herstellen, die für eine jeweilige Pflanzenart typische Wachstumsphasen entsprechen, Dies kann beispielsweise der Fall sein, wenn sich ein erster Trieb ausgebildet hat, sich mindestens eine vorgebbare Anzahl von Trieben ausgebildet haben oder Triebe eine bestimmte Größe erreicht haben.

Zum Drucken sollte ein Kunststoff eingesetzt werden, der einen Röntgenstrahlungsabsorptionswert aufweist, der um maximal ± 50 %, bevorzugt maximal 30 % von Röntgenabsorptionswerten natürlicher Keime abweicht. Dabei können Abweichungen in Richtung höherer Röntgenstrahlungsabsorption besser toleriert werden, als dies bei kleineren Absorptionswerten der Fall wäre. Die o.g. ± - Werte sollten daher eher als maximal 50 % bzw. bevorzugt maximal 30 % gewertet werden. Dazu können einem polymeren Kunststoff auch Additive zugesetzt werden, mit denen eine verbesserte Anpassung der Röntgenabsorptionswerte erreichbar ist.

Mit der Erfindung kann das Problem der schlechten Haltbarkeit von Keimpflanzen umgangen werden.

Es ist ein Druck auch feinster Strukturen bis in die Größenordnung von zehnteln von Millimetern möglich. Es können viele kritische Merkmale eines Keimlings, wie z.B. Wurzeln, Blätter, Stiel oder Samenreste gezielt nachgebildet und auch modifiziert werden, um insbesondere auch typische Anomalien von Keimlingen nachbilden zu können.

Nach dem Drucken künstlicher Keimlinge kann eine Bearbeitung an den künstlichen Keimlingen durchgeführt werden, bei der an der Oberfläche eines Keimlings vorhandene Konturelemente freigelegt oder aufgerichtet werden. Dabei kann überschüssiger Kunststoff mechanisch oder thermisch, z.B. mittels Laserstrahlung entfernt oder auch ein Aufbiegen von Konturelementen, wie z.B. Trieben oder Blättern erfolgen.

Die hergestellten Künstlichen Keimlinge können dann in die gleichen Keimgefäße und mit den gleichen Substraten eingestellt werden, wie sie auch bei den Keimtests mit natürlichen Keimlingen eingesetzt werden. Damit können diese künstlichen Keimlinge den gleichen Prüfprozess durchlaufen, wie bei der Keimlingsprüfung mit natürlichen Keimlingen und die Messergebnisse in einer Regelkarte eingetragen werden.

Der wesentliche Vorteil gegenüber der bisherigen Lösungen besteht darin, dass eine echte Qualitätskontrolle einer röntgentomographischen Prüfeinrichtung mit Regelkarte durchgeführt werden kann.

## Patentansprüche

1. Verfahren zur Überwachung und/oder Kalibrierung einer Einrichtung, die zur dreidimensionalen röntgenoptischen Überprüfung von Keimlingen in verschiedenen Wachstumsphasen ausgebildet ist, bei dem natürliche Keimlinge zu vorgegebenen Zeiten während ihrer Wachstumsphase dreidimensional optisch oder röntgenoptisch vermessen und mit den erfassten Messwerten jeweils ein Steuerungsprogramm für eine Einrichtung, die zum dreidimensionalen Drucken künstlicher Keimlinge als Referenzproben, die den natürlichen Keimlingen nachgebildet sind, ausgebildet ist, erstellt wird und mit der Einrichtung entsprechend den erstellten Steuerprogrammen künstliche Keimlinge mit einem Kunststoff hergestellt werden und
die so hergestellten künstlichen Keimlinge dreidimensional röntgenoptisch vermessen werden und mit den dabei erfassten Messwerten eine Regelkarte eingetragen oder eine bereits erstellte Regelkarte angepasst wird, mit der eine Überwachung und/oder Kalibrierung der Einrichtung, die zur dreidimensionalen röntgenoptischen Überprüfung von Keimlingen ausgebildet ist, durchgeführt wird.

2. Verfahren nach Anspruch 1, **dadurch gekennzeichnet, dass** zum Drucken ein Kunststoff eingesetzt wird, dessen Röntgenstrahlungsabsorptionswert um maximal ± 50 % von Röntgenabsorptionswerten natürlicher Keime abweicht.

3. Verfahren nach einem der vorhergehenden Ansprüche, **dadurch gekennzeichnet, dass** nach dem Drucken künstlicher Keimlinge eine Bearbeitung an den künstlichen Keimlingen durchgeführt wird, bei der an der Oberfläche eines Keimlings vorhanden Komturelemente freigelegt oder aufgerichtet werden.

4. Verfahren nach einem der vorhergehenden Ansprüche, **dadurch gekennzeichnet, dass** beim Drucken typische an natürlichen Keimlingen vorkommende Anomalien nachgebildet werden.

5. Verfahren nach einem der vorhergehenden Ansprüche, **dadurch gekennzeichnet, dass** als Einrichtung, die zur dreidimensionalen röntgenoptischen Überprüfung von Keimlingen ausgebildet ist, ein Computertomograph eingesetzt wird.

## Claims

1. A method for monitoring and/or calibrating a device designed for three-dimensional X-ray optical inspection of seedlings in different growth phases, in which
natural seedlings are optically or X-ray optically measured in three dimensions at predetermined times during their growth phase and a control program for a device which is designed for the three-dimensional printing of artificial seedlings as reference samples which are replicas of the natural seedlings is created in each case using the recorded measured values, and artificial seedlings are produced with a plastic using the device in accordance with the control programs created, and
the artificial seedlings thus produced are measured three-dimensionally by X-ray optics and the measured values thus acquired are recorded in a control chart or an already created control chart is adapted, with which control chart monitoring and/or calibration of the device designed for the three-dimensional X-ray optical inspection of seedlings is performed.

2. The method according to claim 1, **characterized in that**, for printing, a plastic is used, the X-ray absorption value of which deviates by a maximum of ± 50 % from X-ray absorption values of natural seedlings.

3. The method according to any one of the preceding claims, **characterized in that** after printing artificial seedlings, processing is performed on the artificial seedlings in which contour elements present on the surface of a seedling are exposed or raised.

4. The method according to any one of the preceding claims, **characterized in that** typical anomalies occurring on natural seedlings are reproduced during printing.

5. The method according to any one of the preceding claims, **characterized in that** a computer tomograph is used as a device adapted for three-dimensional X-ray optical inspection of seedlings.

## Revendications

1. Procédé de surveillance et/ou d'étalonnage d'un dispositif qui est conçu pour le contrôle tridimensionnel par radiographie de plantules dans différentes phases de croissance, selon lequel
des plantules naturelles sont mesurées optiquement ou radio-graphiquement en trois dimensions à des moments prédéfinis pendant leur phase de croissance et avec les valeurs de mesure acquises, respectivement un programme de commande pour un dispositif, qui est conçu pour l'impression tridimensionnelle de plantules artificielles en tant qu'échantillons de référence, qui sont reproduits à l'image des plantules naturelles, est établi, et avec le dispositif des plantules artificielles sont fabriquées avec une matière plastique conformément aux programmes de commande établis, et
les plantules artificielles ainsi fabriquées sont mesurées par radiographie tridimensionnelle et, avec les valeurs de mesure ainsi acquises, une carte de contrôle est inscrite ou une carte de contrôle déjà établie est adaptée, avec laquelle une surveillance et/ou un étalonnage du dispositif, qui est conçu pour le contrôle tridimensionnel par radiographie de plantules, est effectué.

2. Procédé selon la revendication 1, **caractérisé en ce qu'**une matière plastique, dont la valeur d'absorption des rayons X s'écarte de ± 50 % au maximum des valeurs d'absorption des rayons X de germes naturels, est utilisée pour l'impression.

3. Procédé selon l'une quelconque des revendications précédentes, **caractérisé en ce que**, après l'impression de plantules artificielles, un traitement sur les plantules artificielles est effectué, au cours duquel des éléments de contour présents à la surface d'une plantule sont mis à nu ou redressés.

4. Procédé selon l'une quelconque des revendications précédentes, **caractérisé en ce que**, lors de l'impression, des anomalies typiques présentes sur des plantules naturelles sont reproduites.

5. Procédé selon l'une quelconque des revendications précédentes, **caractérisé en ce qu'**un tomographe assisté par ordinateur est utilisé comme dispositif qui est conçu pour le contrôle tridimensionnel par rayons X de plantules.
